Europäisches Patentamt

European Patent Office

Office européen des brevets

(11) Publication number: **0 112 452**
**B1**

# EUROPEAN PATENT SPECIFICATION

(45) Date of publication of patent specification: 16.12.87

(51) Int. Cl.⁴: **C 07 C 79/04**

(21) Application number: 83110434.4

(22) Date of filing: 19.10.83

(54) Catalytic preparation of nitroalkanes from alkanols.

(30) Priority: 01.12.82 US 446076

(43) Date of publication of application:
04.07.84 Bulletin 84/27

(45) Publication of the grant of the patent:
16.12.87 Bulletin 87/51

(84) Designated Contracting States:
BE CH DE FR GB IT LI LU NL SE

(56) References cited:
US-A-3 957 889
US-A-4 122 124

JOURNAL OF THE AMERICAN CHEMICAL
SOCIETY, vol. 76, no. 10, 26th May 1954, pages
2692-2694; H.B.HASS et al.: "Vapor phase
nitration of aliphatic ethers, alcohols, ketones
and carboxylic acids"

(73) Proprietor: THE DOW CHEMICAL COMPANY
2030 Dow Center Abbott Road P.O. Box 1967
Midland, MI 48640 (US)

(72) Inventor: Hayes, William Verne
202 Hackberry
4403 Clute Texas 77531 (US)

(74) Representative: Weickmann, Heinrich, Dipl.-Ing.
et al
Patentanwälte Dipl.-Ing. H.Weickmann Dipl.-
Phys.Dr. K.Fincke Dipl.-Ing. F.A.Weickmann
Dipl.-Chem. B. Huber Dr.-Ing. H. Liska Dipl.-
Phys.Dr. J. Prechtel Postfach 860820
D-8000 München 86 (DE)

# 0 112 452

**Description**

Nitroalkanes are an essential stabilizing ingredient employed in 1,1,1-trichloroethane when it is used in vapor degreasing and cold cleaning. All manufacturers throughout the world add nitromethane and/or nitroethane to their commercial 1,1,1-trichloroethane-based solvents. Normally nitro-alkanes are manufactured by a vapor phase nitration of the alkane with either nitric acid or $NO_2$. There is a mixture of products formed due to carbon-carbon scission. Thus, for example, when propane is nitrated, the products include 1-nitropropane, 2-nitropropane, nitroethane and nitromethane. Because of the oxidative conditions other oxygen containing compounds are also produced, e.g. aldehydes, acids and carbon oxides. Patents disclosing such a process are U.S. 2,844,634 and 2,905,724.

Improvements in these vapor phase nitrations are claimed by employing the nitric acid or nitrogen oxides together with oxygenated sulfur compounds, e.g. $SO_2$, $H_2SO_4$, (U.S. 3,272,874) and by conducting the nitration in the presence of ozone (U.S. 3,113,975).

Other processes involve nitration of alkanes by nitrogen peroxide $(NO_2)_2$ in the presence of oxygen (air) under pressure at 150°—330°C (U.S. 3,780,115); reacting an alkene with nitric acid in the presence of a lower aliphatic monocarboxylic acid anhydride to produce a nitroester, subsequently reducing it with an alkali borohydride to form the nitroalkane (U.S. 3,706,808) and reacting organic amines with ozone (U.S. 3,377,387).

U.S. 4,122,124 describes a process for the production of trinitromethane by reacting isopropyl alcohol with nitric acid. It is stated that no catalyst is required for said method.

U.S. 3,957,898 is concerned with the nitration of an aromatic or substituted aromatic compound in the presence of nitric acid. The reaction is preferably carried out at room temperature and enhances the selectivity of the nitration at the para position.

The journal of the American Chemical Society Vol. 76, No. 10, (1954), page 2,692 describes the preparation of nitro compounds by a vapor phase of nitration aliphatic ethers, alcohols, ketones and carboxylic acids. According to said method, no catalysts are used.

The present invention is a departure from known methods in that it employs the reaction of an alkanol with nitric acid, or $NO_2$ gas, in the vapor phase over a catalyst.

The catalyst is a salt or an oxide of a Group II metal, e.g. calcium or barium chloride, which may be supported or pelleted.

The process for manufacturing lower nitroalkanes, especially those containing 1—3 carbon atoms, according to the present invention involves a vaporization of a lower alkanol and nitric acid, mixing their vapors and passing over a catalyst which is a salt or oxide of a metal from Group II of the periodic table. The alkanol and nitric acid are conveniently pumped as liquids to individual vaporizers, mixed, and fed to a fixed bed catalyst over which they are transformed into nitromethane. An inert gas, e.g. nitrogen, is preferably used as a diluent, and can be recycled.

The vapors of the alkanol and nitric acid are thoroughly mixed, desirably in proportion of 10 to 1 moles of alkanol per mole of $HNO_3$, preferably 4 to 2, and this mixture is preferably diluted with an inert gas, e.g. nitrogen, desirably at 2 to 15 moles of the inert diluent per mole of alkanol. The preferred range is from about 4 to about 10 moles of diluent per mole of alkanol reactant. The diluent may be selected from inert gases including nitrogen, argon, the carbon oxides, steam and mixtures thereof.

The gas mixture is preferably preheated to a temperature of from 100 to 250°C and the catalyst bed is preferably maintained at a temperature within the range of 150 to 350°C, more preferably from 210 to 260°C.

The catalyst is a compound of metals of Group II of the periodic table, preferably magnesium, calcium, strontium and barium. Salts of these metals, including the chlorides, sulfates, and nitrates may be employed. The oxides of these metals are also useful as catalysts for the reaction. They may be employed separately or in combination. A preferred combination is $CaCl_2/BaCl_2$ in a molar ratio of 1/4 to 4/1.

Either the oxides or salts may be burdened on an inert support and used in this manner. Methods of making supported catalysts are well known to the art. For example, the support may be impregnated by immersing it in a salt solution or by spraying the solution onto the support. A slurry is used in the case of oxides or insoluble salts.

Pressures employed in the process are desirably from 1 to 150 psig (6.9 to 7130 kPa gauge) and preferably from 6 to 50 psig (41.4 to 345 kPa gauge).

Representative preparation of catalyst

A catalyst was made for use in the following Examples by immersing an alumina support (a low surface area (<1 m²/g) spherical support of medium porosity manufactured by Norton and designated SA-5205) in an amount of aqueous $CaCl_2$ solution sufficient to completely wet it. Excess water was evaporated and the catalyst dried. The amount of $CaCl_2$ supplied was sufficient to provide a 21 percent by weight loading on the support. Portions were calcined under a nitrogen purge (oxygen excluded) at 150°C, 415°C, 500°C, 600°C, and 700°C each for a period of 4 hours.

Example 1

The different portions of the above prepared catalysts were run in a 4 foot by 3/4 inch (1220 mm by 19

2

mm) stainless steel tubular reactor system equipped with fluidized sand heat control, pressure and flow controllers, chilled water scrubber column, and an alarm system. A Brooks thermal mass controller was used to meter nitrogen flow. Milton Roy positive displacement pumps were used to meter the methanol and nitric acid flows. Methanol conversion differed only slightly, varying from 13 percent to 22 percent for the reaction run at 245°C, 5.5 sec contact time with a MeOH/HNO$_3$/N$_2$ ratio of 4/1/24. Selectivity varied considerably for the reaction under the above conditions as is shown below in tabular form.

| Calcination temp. (°C) | % Selectivity to CH$_3$NO$_2$ |
|---|---|
| 150 | 27 |
| 415 | 35 |
| 500 | 40 |
| 600 | 44 |
| 700 | 67 |

Thus, the preferred method of preparing the catalyst is to calcine the salt or oxide of the metal on a support for a period of 2 to 10 hours at a temperature of from 500° to 700°C.

Example 2

A quantity (370 ml.) of a catalyst prepared according to the above description, calcined at 700°C for 4 hours and consisting of a 1/1 (atomic ratio of Ca/Ba) mixture of calcium chloride and barium chloride coated at 17.6 percent by weight on a low surface area alumina support (SA-5205) was loaded into the 4-foot (1220 mm) stainless steel tube reactor described above and heated to 270°C with a diluent gas (nitrogen) purging through the system. The pressure was controlled at 7 psig, preheater temperature 185°C, nitrogen flow 4000 cc/min., then nitric acid was started at 0.0076 gram mole/min. rate. Methanol was then started at 0.0301 gram mole/min. rate (4/1/24 CH$_3$OH/HNO$_3$/N$_2$ mole ratio).

Analysis of the condensed reactor effluent showed a 17 percent conversion of methanol and a 60 percent selectivity to nitromethane.

Examples 3—7

A supported catalyst containing CaCl$_2$/CaO (1/1 mole ratio) was employed at different reactant ratios, contact times and temperatures to obtain the conversions and selectivities shown in Table I. The support was the same as employed in Examples 1 and 2 above and catalyst loading was 19.7 percent based on weight of the finished catalyst.

TABLE I

| Ex. No. | Temp. (°C) | CH$_3$OH/HNO$_3$/N$_2$ ratio | Contact time (sec.) | Methanol conversion (%) | Nitromethane selectivity (%) |
|---|---|---|---|---|---|
| 3 | 263 | 4.74/1/24 | 7.0 | 16.1 | 57.1 |
| 4 | 250 | 4.73/1/24 | 4.6 | 11.9 | 63.5 |
| 5 | 256 | 3.16/1/24 | 3.6 | 18.7 | 53.0 |
| 6 | 255 | 4/1/24 | 3.6 | 21.7 | 45.7 |
| 7 | 284 | 4/1/24 | 6.2 | 17.0 | 71.5 |

# 0 112 452

Examples 8—14

In other experiments anhydrous $CaCl_2$ pellets (5 mesh) were used with conditions and results shown in Table II.

## TABLE II

| Ex. No. | Pressure (psig) | Bed temp. (°C) | MEOH/HNO$_3$/N$_2$ ratio | Contact time (sec.) | Methanol conversion (%) | Selectivity to nitro-methane (%) |
|---|---|---|---|---|---|---|
| 8 | 8.5 | 285 | 1.58/1/24 | 2.61 | 28.5 | 29.5 |
| 9 | " | " | " | 2.15 | 22.0 | 37.6 |
| 10 | " | " | " | 1.83 | 25.2 | 30.4 |
| 11 | " | " | 2.37/1/24 | 2.69 | 17.1 | 48.5 |
| 12 | 7.0 | 342 | 4.39/1/24 | 1.00 | 22.0 | 31.7 |
| 13 | " | 332 | " | 1.16 | 30.1 | 26.6 |
| 14 | " | " | " | " | 30.9 | 24.5 |

Examples 15—21

Other Group II metals were tested in a 1-foot by 1 inch (305 mm×25.4 mm) stainless steel tubular reactor equipped as the longer reactor. Conditions under which the reaction was run were pre-heater temperature 185°C, nitrogen flow 4000 ml/min. and pressure 7 psig. Table III shows the reaction parameters and results:

## TABLE III

| Ex. No. | Temp. (°C) | Catalyst composition | Contact time (sec.) | CH$_3$OH/HNO$_3$/N$_2$ mole ratio | CH$_3$OH conv. (%) | CH$_3$NO$_2$ select. (%) | Wt.* % Load. |
|---|---|---|---|---|---|---|---|
| 15 | 215 | BaCl$_2$ | 3.7 | 4/1/24 | 12.0 | 64.6 | 18 |
| 16 | 275 | MgCl$_2$ | 4.5 | 3.8/1/24 | 32.7 | 26.5 | 17.7 |
| 17 | 230 | SrCl$_2$ | 4.2 | 3.7/1/24 | 20.8 | 40.9 | 15.5 |
| 18 | " | CaCl$_2$/BaCl$_2$ | 3.5 | 4/1/24 | 17.0 | 60.0 | 17.6 |
| 19 | 200 | (BaCl$_2$)$_2$/CaCl$_2$ | 3.4 | 4/1/24 | 33.0 | 28.0 | 21.3 |
| 20 | 120 | (CaF$_2$)$_9$/CaCl$_2$ | 3.4 | 4/1/24 | 23.0 | 19.0 | 20.7 |
| 21 | 230 | CaBr$_2$ | 5.2 | 3.9/1/24 | 14.0 | 23.0 | 19.7 |

* Catalyst loading is based on total weight of salt and support (low surface alumina).

Example 22

A catalyst of 16 percent $CaCl_2$ on alumina spheres, calcined at 700°C for 4 hours, employed in the 4-foot (1220 mm) reactor at various temperatures, was run at a MeOH/HNO$_3$/N$_2$ ratio of 3.8/1/24, 4 sec. contact time and a pressure of 8 psig. A quantity of 370 ml of catalyst was employed as in Example 1. Results are shown in Table IV.

**0 112 452**

## TABLE IV

| Temp. (°C) | % Methanol conversion | % Selectivity to nitromethane |
|---|---|---|
| 245 | 8.3 | 68.0 |
|  | 9.7 | 60.5 |
| 260 | 11.6 | 53.1 |
|  | 10.0 | 63.1 |
| 272 | 14.6 | 47.7 |
|  | 16.3 | 45.3 |

## Claims

1. A process for making nitroalkanes by reacting in the vapor phase a mixture of a lower alkanol and nitric acid or nitrogen dioxide, the improvement of which comprises the reaction in the presence of a catalyst which is an oxide or a salt of at least one metal of Group II of the periodic table.

2. The process of Claim 1 wherein the catalyst is an oxide or salt of calcium, barium, strontium or magnesium.

3. The process of Claim 2 wherein an inert diluent gas is employed and the reactant and diluent gases are preheated.

4. The process of Claim 3 wherein the gases are preheated to a temperature of from 100 to 250°C and the reaction temperature is from 150 to 350°C.

5. The process of Claim 4 wherein the molar ratio of lower alkanol to nitric acid is from 10/1 to 1/1.

6. The process of Claim 5 wherein the inert diluent gas is present in an amount of from 2 to 15 moles based on moles of alkanol present.

7. The process of Claim 1 wherein the catalyst is an oxide or salt of calcium and barium in combination.

8. The process of Claim 1 wherein the lower alkanol contains 1—3 carbons.

9. The process of Claim 8 wherein the lower alkanol is methanol or ethanol.

10. The process of Claim 9 wherein the inert diluent gas is nitrogen, argon, CO, $CO_2$, steam or mixtures thereof.

11. The process of Claim 10 wherein the temperature of reaction is from 210 to 260°C.

12. The process of Claim 11 wherein the catalyst has been prepared by calcining at a temperature of from 500 to 700°C for a period of 2 to 10 hours.

13. The process of Claim 7 wherein the molar ratio of calcium chloride to barium chloride is from 1/4 to 4/1.

## Patentansprüche

1. Verfahren zur Herstellung von Nitroalkanen durch Umsetzung einer Mischung eines niederen Alkanols und Salpetersäure oder Stickstoffdioxid in der Dampfphase, dadurch gekennzeichnet, daß die Umsetzung in Gegenwart eines Katalysators durchgeführt wird, der ein Oxid oder ein Salz von mindestens einem Metall der Gruppe II des periodischen Systems ist.

2. Verfahren nach Anspruch 1, dadurch gekennzeichnet, daß der Katalysator ein Oxid oder Salz von Calcium, Barium, Strontium oder Magnesium ist.

3. Verfahren nach Anspruch 2, dadurch gekennzeichnet, daß ein inertes Verdünnungsgas verwendet wird und der Reaktionspartner und die Verdünnungsgase vorerhitzt werden.

4. Verfahren nach Anspruch 3, dadurch gekennzeichnet, daß die Gase auf einer Temperatur von 100 bis 250°C vorerhitzt werden, und die Reaktionstemperatur 150 bis 350°C beträgt.

5. Verfahren nach Anspruch 4, dadurch gekennzeichnet, daß das Molverhältnis von niederem Alkanol zu Salpetersäure 10/1 bis 1/1 beträgt.

6. Verfahren nach Anspruch 5, dadurch gekennzeichnet, daß das inerte Verdünnungsgas in einer Menge von 2 bis 15 Mol, bezogen auf die Mole des vorhandenen Alkanols, vorliegt.

7. Verfahren nach Anspruch 1, dadurch gekennzeichnet, daß der Katalysator eine Kombination eines Oxids oder Salzes von Calcium und Barium ist.

8. Verfahren nach Anspruch 1, dadurch gekennzeichnet, daß das niedere Alkanol 1 bis 3 Kohlenstoffatome enthält.

9. Verfahren nach Anspruch 8, dadurch gekennzeichnet, daß das niedere Alkanol Methanol oder Ethanol ist.

10. Verfahren nach Anspruch 9, dadurch gekennzeichnet, daß das inerte Verdünngsgas Stickstoff, Argon, CO, $CO_2$, Wasserdampf oder eine Mischung davon ist.

11. Verfahren nach Anspruch 10, dadurch gekennzeichnet, daß die Reaktionstemperatur 210 bis 260°C beträgt.

5

12. Verfahren nach Anspruch 11, dadurch gekennzeichnet, daß der Katalysator durch Calcinieren bei einer Temperatur von 500 bis 700°C während einer Dauer von 2 bis 10 Stunden hergestellt wurde.

13. Verfahren nach Anspruch 7, dadurch gekennzeichnet, daß das Molverhältnis von Calciumchlorid zu Bariumchlorid 1/4 bis 4/1 ist.

## Revendications

1. Procédé de production de nitro-alcanes, par réaction en phase vapeur d'un mélange d'un alcanol inférieur et d'acide nitrique ou de dioxyde d'azote, perfectionné en ce qu'on effectue la réaction en présence d'un catalyseur qui est un oxyde ou un sel d'au moins un métal du Groupe II de la Classification Périodique.

2. Procédé selon la revendication 1, dans lequel le catalyseur est un oxyde ou un sel de calcium, baryum, strontium ou magnésium.

3. Procédé selon la revendication 2, dans lequel on utilise un diluant inerte gazeux et on préchauffe le réactif et le diluant gazeux.

4. Procédé selon la revendication 3, dans lequel on préchauffe les gaz à une température comprise entre 100 et 250°C, et la température de réaction est comprise entre 150 et 350°C.

5. Procédé selon la revendication 4, dans lequel le rapport molaire de l'alcanol inférieur à l'acide nitrique est compris entre 10/1 et 1/1.

6. Procédé selon la revendication 5, dans lequel le diluant gazeux inerte est présent à raison de 2 à 15 moles par mole de l'alcanol présent.

7. Procédé selon la revendication 1, dans lequel le catalyseur est une combinaison d'oxydes ou de sels de calcium et de baryum.

8. Procédé selon la revendication 1, dans lequel l'alcanol inférieur comporte de 1 à 3 atomes de carbone.

9. Procédé selon la revendication 8, dans lequel l'alcanol inférieur est le méthanol ou l'éthanol.

10. Procédé selon la revendication 9, dans lequel le diluent gazeux inerte est l'azote, l'argon, CO, $CO_2$, la vapeur d'eau ou des mélanges de ceux-ci.

11. Procédé selon la revendication 10, dans lequel la température de la réaction est comprise entre 210 et 260°C.

12. Procédé selon la revendication 11, dans lequel on prépare le catalyseur par calcination à une température comprise entre 500 et 700°C pendant une durée de 2 à 10 heures.

13. Procédé selon la revendication 7, dans lequel le rapport molaire du chlorure de calcium au chlorure de baryum est de 1/4 à 4/1. .